# EUROPÄISCHE PATENTANMELDUNG

(11) **EP 2 695 652 A1**
(43) Veröffentlichungstag der Anmeldung: **12.02.2014**
(21) Anmeldenummer: 12179895.3
(22) Anmeldetag: 09.08.2012
(51) Int. Cl.: B01D 1/00, G01N 33/49, B01L 3/00

(54) **System zur Abtrennung von Plasma aus Vollblut**

(71) Anmelder: F. Hoffmann-La Roche AG, 4070 Basel (CH); Roche Diagnostics GmbH, 68305 Mannheim (DE)
(72) Erfinder: Huemer, Herfried, 8330 Feldbach (AT)
(74) Vertreter: Babeluk, Michael

(57) **Zusammenfassung**

Die Erfindung betrifft ein System zur Abtrennung von Plasma aus Vollblut, mit einem Sammelbehälter (2) zur Aufnahme von Vollblut (41), mit einer Filtereinrichtung (1) zur Plasmagewinnung, die eingangsseitig an den Sammelbehälter (2) anschließbar ist, sowie mit einer Fördereinheit, die eine Unterdruck erzeugende Pumpe (9) aufweist. Erfindungsgemäß ist die Fördereinheit Teil eines Analysators, wobei die Filtereinrichtung (1) mit dem angeschlossenen Sammelbehälter (2) ausgangsseitig der Filtereinrichtung (1) an ein Probeneingabeteil (8) des Analysators andockbar ist.

## Beschreibung

Die Erfindung betrifft ein System zur Abtrennung von Plasma aus Vollblut, mit einem Sammelbehälter zur Aufnahme von Vollblut, mit einer Filtereinrichtung zur Plasmagewinnung, die eingangsseitig an den Sammelbehälter anschließbar ist, sowie mit einer Fördereinheit, die eine Unterdruck erzeugende Pumpe aufweist. Die Erfindung betrifft weiters eine Filtereinrichtung sowie ein Verfahren zur Abtrennung von Plasma aus Vollblut.

Neben den vor allem in Labors verwendeten Zentrifugen zur Plasmaabtrennung sind bereits eine Reihe von Vorrichtungen bekannt geworden, mit welchen auch im dezentralen Point-of-Care (PoC)-Bereich kleine Plasmamengen durch Abtrennung aus Vollblut mittels Filtern zur Verfügung gestellt werden.

Im einfachsten Fall kann die Plasmaabtrennung durch einen mehrschichtigen Teststreifen gemäß DE 40 15 589 A1 (BOEHRINGER MANNHEIM) erfolgen, welcher auf einer inerten Trägerfolie eine Transportschicht zum Transport der Probenflüssigkeit (Vollblut) von einem Aufgabebereich in einen Messbereich aufweist. Die Transportschicht kann beispielsweise aus einem Glasfaservlies bestehen, das im Aufgabebereich von einer Plasmaabtrennschicht bedeckt ist.

Aus der EP 0 550 950 A2 (SANWA KAGAKU KENKYUSHO) ist ein Verfahren und eine Vorrichtung zur Separation von Blutserum und Plasma bekannt. In diesem Dokument werden unterschiedliche Ausführungsvarianten von Vorrichtungen zur Plasmagewinnung dargestellt, wobei beispielsweise in den Figuren 1 bis 4 Varianten offenbart sind, bei welchen eine Plasmaabtrennvorrichtung in eine Blutgewinnungseinrichtung integriert ist. Das Blut wird zunächst mittels Unterdruck in einen Sammelbehälter gesaugt, in welchem ein zweischichtiges Trennfilter angeordnet ist. Nach Abschluss der Blutentnahme wird der Sammelbehälter mit einem evakuierten Fluidbehälter verbunden, wobei das Plasma durch das Trennfilter abgetrennt und im Fluidbehälter gesammelt wird. Bei einer Ausführungsvariante gemäß Fig. 5 und Fig. 6 wird der für die Plasmaabtrennung erforderliche Unterdruck durch eine Kolbenspritze erzeugt. Weiters zeigt die Ausführungsvariante gemäß Figuren 9 und 10 eine Art Spritzenvorsatzfilter, welches ebenfalls zur Plasmagewinnung verwendet werden kann.

Aus der WO 2011/033000 A2 (F. HOFFMANN LA ROCHE AG) ist eine mehrteilige Probeneingabevorrichtung zur Eingabe von flüssigen Proben in einen Analysator bekannt geworden. Die Eingabevorrichtung weist Anschlüsse zur Herstellung einer Verbindung zwischen einem die Probe aufnehmenden Sammelbehälter (z.B. Kolbenspritze) und dem Einfüllmund eines Analysators auf. Ein Analysator-Verbindungsteil der Eingabevorrichtung weist in seinem Inneren ein Rückhalteelement, beispielsweise ein Gitter, auf, das partikuläre Bestandteile der Probe vom Übertritt in den Analysator zurückhält. Ein Probengefäß-Verbindungsteil der Eingabevorrichtung, das mit dem Analysator-Verbindungsteil durch eine Schnappverbindung unlösbar verbunden ist, weist ein Einsaugrohr auf, welches in das Innere der Kolbenspritze ragt, wobei der als Luer-Konus ausgebildete Anschluss des Probengefäß-Verbindungsteils Belüftungskanäle aufweist, um während der Probenentnahme Luft in das Innere der Kolbenspritze zu führen.

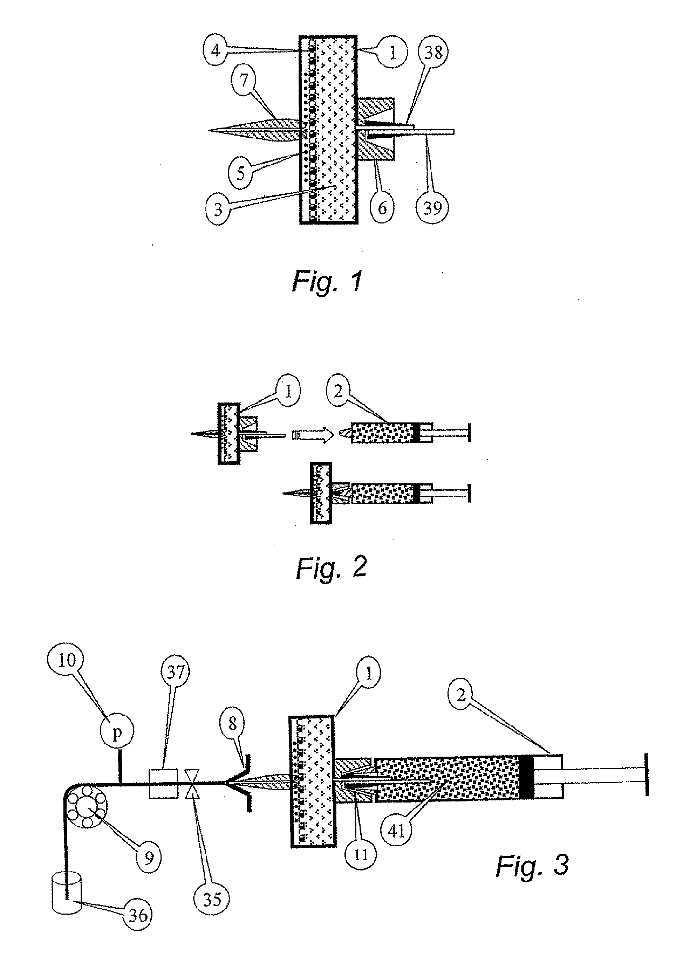

Aus der WO 96/24425 A1 (FIRST MEDICAL INC.), insbesondere deren Fig. 1 bis Fig. 3 und Fig. 8, ist eine Vorrichtung und ein Verfahren zur Plasmaabtrennung bekannt. Eine 'Blood Separation Device' genannte Einrichtung weist ein Filterelement, einen flexiblen Schlauch und an dessen Ende eine Nadel auf, welche in ein 'Blood Collection Device' eingeführt wird. Mit einer Antriebseinheit, die eine auf den flexiblen Schlauch einwirkende Schlauchpumpe aufweist, wird Vollblut aus dem 'Blood Collection Device' angesaugt und durch das Filterelement gepumpt, wobei Plasma separiert wird und an einer Plasma-Auslassöffnung der Filtereinheit für die weitere Verwendung zur Verfügung steht. Nachteilig sind die ungeregelten, relativ großen Druckwerte, die im Druckbetrieb vor der Filtereinheit auftreten, sowie der im Entnahmegefäß bei fortgesetzter Entnahme von Vollblut auftretende Unterdruck.

Aufgabe der Erfindung ist es, ein System zur Abtrennung von Plasma aus Vollblut, eine entsprechende Filtereinrichtung sowie ein Verfahren zur Abtrennung von Plasma aus Vollblut vorzuschlagen, welches einfach und kostengünstig zu handhaben ist, wobei auch bei geringen Probenmengen und/oder hohen Hämatokritwerten reproduzierbar Plasmaproben gewonnen werden können.

Die erfindungsgemäße Lösung sieht ausgehend von der WO 96/24425 A1 ein System vor, bei dem die Fördereinheit Teil eines Analysators ist und die Filtereinrichtung mit dem angeschlossenen Sammelbehälter ausgangsseitig der Filtereinrichtung an ein Probeneingabeteil des Analysators angedockt wird.

Bevorzugt weist die Fördereinheit eine Regeleinrichtung zur Einstellung eines maximal zulässigen Unterdrucks in der Filtereinheit auf, welche zur Regelung der Förderleistung der den Unterdruck erzeugenden Pumpe, beispielsweise einer Schlauchpumpe, dient.

Die Filtereinheit weist eingangsseitig (d.h. auf der Seite, an welche der Vollblut enthaltende Sammelbehälter angeschlossen werden kann) ein Entnahmerohr und ein Belüftungsrohr auf, die beide in den Sammelbehälter (z.B. Spritze mit Luer-Anschluss) eingeführt werden, so dass bei der Entnahme von Vollblut kein störender Unterdruck im Sammelbehälter auftritt.

Um das Ansaugen von Luftbläschen zu vermeiden, ragt das Belüftungsrohr bevorzugt weiter in den Sammelbehälter als das Entnahmerohr, d.h. der in den Sammelbehälter ragende Teil des Belüftungsrohres ist länger als der in den Sammelbehälter ragende Teil des Entnahmerohres.

Das erfindungsgemäße Verfahren zur Abtrennung von Plasma aus Vollblut ist durch folgende Schritte gekennzeichnet:
- Bereitstellen eines mit Vollblut befüllten Sammelbehälters;
- Ankoppeln des mit Vollblut befüllten Sammelbehälters an eine Filtereinrichtung, bevorzugt durch Einführen eines Entnahmerohrs und eines Belüftungsrohrs der Filtereinrichtung in den Sammelbehälter;
- Andocken der Filtereinrichtung an das Probeneingabeteil eines Analysators;
- Starten einer mit dem Probeneingabeteil verbundenen, Unterdruck erzeugenden Pumpe (Saugpumpe) des Analysators, so dass ausgangsseitig (d.h. auf der dem Probeneingabeteil des Analysators zugewandten Seite der Filtereinrichtung) aus der Filtereinrichtung Plasma austritt; und
- Bereitstellen des so gewonnenen Plasmas zur Analytbestimmung im Analysator.

Bevorzugt kann der Unterdruck in der Filtereinheit durch eine Regeleinrichtung des Analysators, vorzugsweise durch eine druckabhängige Steuerung der Förderleistung der Saugpumpe, geregelt werden.

Die Erfindung wird im Folgenden anhand von Zeichnungen näher erläutert. Es zeigen:
- Fig. 1: eine erfindungsgemäße Filtereinrichtung zur Abtrennung von Plasma aus Vollblut in einer Schnittdarstellung;
- Fig. 2: eine Verpackungseinheit aus einer Filtereinrichtung gemäß Fig. 1 und einem Sammelbehälter (Kolbenspritze mit Luer-Anschluss) getrennt und zusammengesetzt;
- Fig. 3: ein erfindungsgemäßes System zur Abtrennung von Plasma aus Vollblut in einer schematischen Darstellung; sowie die

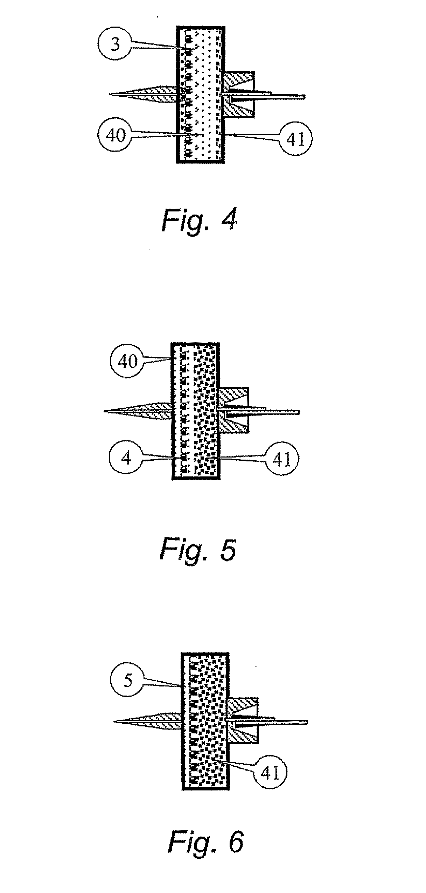

- Fig. 4: bis Fig. 6 die Filtereinrichtung gemäß Fig. 1 in unterschiedlichen Stadien der Ausbildung einer Plasmafront.

Die in den Fig. 1 bis Fig. 3 dargestellte Filtereinrichtung 1 zur Abtrennung von Plasma aus Vollblut 41, das in einem Sammelbehälter 2 vorliegt, weist in einem Filtergehäuse ein Schichtfilter, beispielsweise bestehend aus Tiefenfilter 3, Stoppmembran 4 und Lateralgitter 5, auf, wobei eingangsseitig eine Aufnahme 6 mit Belüftungsöffnung, beispielsweise ein belüfteter Luer-Kegel, für den Anschluss des Sammelbehälters 2 (beispielsweise eine Kolbenspritze mit Luer-Konus) und ausgangsseitig ein Kapillaradapter 7 zum Anschluss an das Probeneingabeteil 8 eines Analysators vorgesehen sind.

Das in Fig. 3 schematisch dargestellte Gesamtsystem zur Abtrennung von Plasma aus Vollblut bedient sich einer Fördereinheit eines nicht weiter dargestellten Analysators, welche eine Pumpe 9 aufweist, die den für die Plasmaabtrennung erforderlichen Unterdruck erzeugt. Die Fördereinheit weist eine nicht weiter dargestellte Regeleinrichtung zur Regelung der Förderleistung der Pumpe 9 auf, mit welcher ein vorgebbarer Unterdruck in der Filtereinheit 1 eingestellt werden kann. Dafür weist die Regeleinrichtung einen Drucksensor 10 auf, dessen Ausgangssignal mit der Steuereinheit der Pumpe 9 (beispielsweise einer Schlauchpumpe) verbunden ist, die ausgangsseitig mit einem Abfallbehälter 36 verbunden ist.

Bei der Plasmagewinnung mit dem System gemäß Fig. 3 kann beispielsweise wie folgt vorgegangen werden:
- Entnahme einer Filtereinrichtung 1 (im Detail dargestellt in Fig. 1) aus einer sterilen Verpackung.
- Andocken des belüfteten Luer-Kegels 6 der Filtereinrichtung 1 an einen mit mindestens 500 µl, besser 1 ml, Vollblut 41 gefüllten Sammelbehälter 2, z.B. einer 2ml-Kolbenspritze, gemäß Fig. 2.
- Fig. 3: Andocken des Kapillaradapters 7 an das Probeneingabeteil 8 eines nicht weiter dargestellten Analysators.
- Anwählen der Auswahl "Kapillarmessung" am Analysator (z.B. cobas b 221 von Roche Diagnostics).
   *(Alternativ kann der ausgangsseitige Adapter an der Filtereinrichtung 1 auch als Luer-Anschluss ausgeführt sein. In diesem Fall ist beispielsweise der Modus "Spritzenmessung" am Analysator anzuwählen und erst danach kann die Filtereinrichtung mit dem aufgesteckten Sammelbehälter 2 angedockt werden).*
- Saugbeginn bei Kapillarmessung: Die Pumpe 9 der Fördereinrichtung des Analysators, beispielsweise eine Schlauchpumpe, wird aktiviert und beginnt sich im Falle einer Schlauchpumpe zu drehen, so dass an der ausgangsseitigen Seite der Filtereinrichtung 1 ein Unterdruck aufgebaut wird.
   *(Alternativ kann die Pumpe 9 im Modus "Spritzenmessung" manuell gestartet werden, sobald die Filtereinrichtung 1 am Probeneingabeteil 8 des Analysators angedockt ist).*
- Mit dem Drucksensor 10 der Regeleinrichtung des Analysators kann die Förderleistung der Pumpe 9 so geregelt werden, dass sich in der Filtereinheit 1 ein Unterdruck von max. 500 mbar, besser 300 mbar, ideal 100 bis 150 mbar, einstellt.
   Der belüftete, doppellumige Luer-Adapter 6 (mit dem Entnahmerohr 38 und dem Belüftungsrohr 39) ermöglicht ein Saugen aus der Spritze unter gleichzeitigem Druckausgleich. Dazu können an der kegelförmigen Oberfläche des Luer-Adapters 6 der Filtereinrichtung 1 nutartige Kanäle 11 ausgebildet sein oder eine gasdurchlässige, vorzugsweise hydrophobe Schicht vorliegen, die für den Druckausgleich sorgen. Das Belüftungsrohr 39 ragt dabei weiter in die Kolbenspritze hinein als das Entnahmerohr 38 und verhindert somit ein Rücksaugen der einströmenden Luftblasen, da im Betriebsmodus einströmende Luftblasen nach oben und somit aus dem Ansaugbereich des Entnahmerohr 38 steigen. Der Luer-Adapter 6 schließt am Luer-Anschluss der Kolbenspritze dicht mit der Innenwand ab.
   Weiterhin hat die Position des Belüftungsrohrs 39 auch Einfluß auf die gewinnbare Probenmenge, da das in den Sammelbehälter ragende Ende des Belüftungsrohres 39 einen "Anschlag" für den Kolben des Sammelbehälters bildet.
   *(Die Belüftung des Luer-Adapters 6 kann alternativ auch mittels porösen, luftdurchlässigen Kunststoffen erfolgen.)*
   Das Tiefenfilter 3 der Filtereinrichtung 1 kann aus bindemittelfreien Glasfasern (typ. FV-2, Fa. Whatman bzw. DE 40 15 589 A1 bzw. EP 0 239 002 A1 Böhringer-Mannheim) mit Rückhaltebereich von 0,5 µm bis 10 µm, besser 1 µm bis 5 µm, vorzugsweise < 3 µm aufgebaut sein. Die roten Blutkörperchen (RBK) lagern sich dann an den feinen Glasfasern des Tiefenfilters 3 an, ohne die Flussrate übermäßig zu beeinflussen bzw. zu zerplatzen (siehe Fig. 4).
- Abhängig vom Querschnitt der Filtereinrichtung 1 und vom Hämatokrit bildet sich eine "Plasmafront" oder "Plasmafraktion" 40 aus, die ungehindert durch die Stoppmembran 4 treten kann. Dabei werden durch die "engmaschige" Stoppmembran 4 die restlichen, vereinzelt vorhandenen RBKs herausgefiltert. (Fig. 5), welche durch den Tiefenfilter nicht zurückgehalten wurden. Hierzu weist die Stoppmembran 4 in Vergleich zum Tiefenfilter 3 eine deutlich geringere Porengröße auf, beispielsweise Poren mit einem Durchmesser von weniger als 400 nm, bevorzugt mit einem Durchmesser von weniger als 200 nm. Durch die Kombination eines Tiefenfilters 3, welcher aufgrund seiner Porengröße bereits den ganz überwiegenden Teil der Blutzellen zurückhält, aber die Plasmafraktion weitgehend ungestört durchfließen lässt, mit einer nachgeschalteten Stoppmembran 4, welche aufgrund ihrer kleineren Porengröße auch die verbleibenden Blutzellen zuverlässig zurückhält, aber aufgrund ihrer begrenzten Porenzahl ohne den vorgeschalteten Tiefenfilter 4 sehr rasch verstopfen würde, kann eine zuverlässige Abtrennung der Blutzellen ohne rasches Verstopfen des Filters erzielt werden, wodurch die Gewinnung eines ausreichenden Volumens einer Plasmaprobe ermöglicht wird.
   Der mit Hilfe des Drucksensors 10 und der geregelten Pumpe 9 eingestellte Unterdruck bestimmt mit den geometrischen Verhältnissen der Filtereinrichtung die Flussrate und somit die Scherkräfte, die in der Stoppmembran 4 speziell auf die RBKs einwirken. Ein Zerplatzen der RBKs (Hämolyse) wird durch den erfindungsgemäßen geregelten Saugbetrieb mit relativ geringer und gleichmäßiger Applikation eines Unterdrucks ohne größere Druckschwankungen wirksam verhindert.
- Fig. 6: Das Lateralgitter 5 ermöglicht ein Sammeln und Absaugen des Plasmas nach der Stoppmembran 4 in Richtung Kapillaradapter 7, indem ein "Abdichten" durch die Stoppmembran 4 verhindert wird. Das Lateralgitter 5 bildet durch seine Gitterstruktur einerseits eine nicht durchgängige Auflage für die Stoppmembran 4, so dass Plasma auf der ausgangsseitigen Seite der Stoppmembran 4 ausfließen kann. Die Gitterstruktur bewirkt durch die Bildung von Kanälen weiterhin, dass das flächig aus der Stoppmembran 4 hindurchtretende Plasma im Bereich des Kapillaradapters 7 zusammenfließen und durch diesen an den nachgeschalteten Analysator abgegeben werden kann.
   *(Diese Funktion des Lateralgitters 5 kann alternativ auch durch Prägen des Bodens der Filtereinrichtung 1 bzw. durch ausreichende Rauhigkeit dessen Oberfläche sichergestellt werden).*
- Nach dem Einsaugen der gewünschten Plasmamenge (beispielsweise detektiert mit einem Sample Sensor 35 eingangs der Messkammer 37) wird durch reversiblen Betrieb der Schlauchpumpe 9 - gesteuert vom Drucksensor 10 - ein Druckausgleich hergestellt, um beim Abdocken der Filtereinrichtung 1 ein Fraktionieren der eingesaugten Plasmamenge zu verhindern.
   *(Alternativ kann die Plasmagewinnung auch bei vorzeitigem Druckanstieg (detektiert mit dem Drucksensor 10) beendet werden, um eine Hämolysierung und damit verbundener Verunreinigung des gewonnenen Plasmas bei hohem Hämatokritwerten und*/*oder geringer Probenmenge zu verhindern.)*
- Abstecken der Filtereinrichtung 1 samt Sammelbehälter 2 vom Probeneingabeteil 8 des Analysators.
- Probenpositionierung im Analysator und analytische Bestimmung, beispielsweise der Hämoglobinwerte im gewonnenen Plasma, in der Messkammer 37 (z.B. Oxymeter).

Einsaugzeit, erwünschte Probenmenge und Filterabmessungen sind voneinander abhängig und können vom Fachmann so gewählt werden, dass eine erfindungsgemäße Plasmagewinnung möglichst ohne zusätzliche Hämolyse erfolgen kann.

## Patentansprüche

1. System zur Abtrennung von Plasma aus Vollblut, mit einem Sammelbehälter (2) zur Aufnahme von Vollblut (41), mit einer Filtereinrichtung (1) zur Plasmagewinnung, die eingangsseitig an den Sammelbehälter (2) anschließbar ist, sowie mit einer Fördereinheit, die eine Unterdruck erzeugende Pumpe (9) aufweist, **dadurch gekennzeichnet, dass** die Fördereinheit Teil eines Analysators ist und die Filtereinrichtung (1) mit dem angeschlossenen Sammelbehälter (2) ausgangsseitig der Filtereinrichtung (1) an ein Probeneingabeteil (8) des Analysators andockbar ist.

2. System nach Anspruch 1, **dadurch gekennzeichnet, dass** die Filtereinrichtung (1) ein Entnahmerohr (38) aufweist, das zusammen mit einem Belüftungsrohr (39) in den Sammelbehälter (2) einführbar ist.

3. System nach Anspruch 2, **dadurch gekennzeichnet, dass** das Belüftungsrohr (39) weiter in den Sammelbehälter (2) ragt als das Entnahmerohr (38).

4. System nach einem der Ansprüche 1 bis 3, **dadurch gekennzeichnet, dass** die Fördereinheit zur Einstellung eines maximal zulässigen Unterdrucks in der Filtereinheit (1) eine Regeleinrichtung zur Regelung der Förderleistung der Unterdruck erzeugenden Pumpe (9) aufweist.

5. System nach Anspruch 4, **dadurch gekennzeichnet, dass** die Regeleinrichtung einen Drucksensor (10) aufweist, dessen Ausgangssignal mit der Steuereinheit der Pumpe (9) verbunden ist.

6. Filtereinrichtung (1) zur Abtrennung von Plasma aus Vollblut, **dadurch gekennzeichnet, dass** die Filtereinrichtung (1) eingangsseitig ein Entnahmerohr (38) und ein Belüftungsrohr (39) aufweist, die in einen Sammelbehälter (2), beispielsweise eine Spritze mit Luer-Anschluss, einführbar sind.

7. Filtereinrichtung (1) nach Anspruch 6, **dadurch gekennzeichnet, dass** das Belüftungsrohr (39) weiter in den Sammelbehälter (2) ragt als das Entnahmerohr (38).

8. Filtereinrichtung (1) nach Anspruch 6 oder 7, **dadurch gekennzeichnet, dass** die Filtereinrichtung (1) eingangsseitig eine Aufnahme mit Belüftungsöffnung, beispielsweise einen belüfteten Luer-Kegel (6), für den Sammelbehälter (2) und ausgangsseitig einen Kapillaradapter (7) zum Anschluss an das Probeneingabeteil (8) eines Analysators aufweist.

9. Filtereinrichtung (1) nach einem der Ansprüche 6 bis 8, **dadurch gekennzeichnet, dass** die Filtereinrichtung (1) in einem Filtergehäuse einen Schichtfilter, beispielsweise bestehend aus Tiefenfilter (3), Stoppmembran (4) und Lateralgitter (5), aufweist.

10. Verfahren zur Abtrennung von Plasma aus Vollblut mit einer Filtereinrichtung (1), **gekennzeichnet durch**
- Bereitstellen eines mit Vollblut (41) befüllten Sammelbehälters (2);
- Ankoppeln des mit Vollblut (41) befüllten Sammelbehälters (2) an eine Filtereinrichtung (1), bevorzugt **durch** Einführen eines Entnahmerohrs (38) und eines Belüftungsrohrs (39) der Filtereinrichtung (1) in den Sammelbehälter (2);
- Andocken der Filtereinrichtung (1) an das Probeneingabeteil (8) eines Analysators;
- Starten einer mit dem Probeneingabeteil (8) verbundenen, Unterdruck erzeugenden Pumpe (9) des Analysators, so dass ausgangsseitig aus der Filtereinrichtung (1) Plasma austritt; und
- Bereitstellen des so gewonnenen Plasmas zur Analytbestimmung im Analysator.

11. Verfahren nach Anspruch 10, **dadurch gekennzeichnet, dass** der Unterdruck in der Filtereinheit (1) durch eine Regeleinrichtung des Analysators, vorzugsweise durch eine druckabhängige Steuerung der Förderleistung der Saugpumpe (9), geregelt wird.

12. Verfahren nach Anspruch 11, **dadurch gekennzeichnet, dass** in der Filtereinheit (1) ein Unterdruck < 500 mbar, bevorzugt < 300 mbar, besonders bevorzugt zwischen 100 und 150 mbar, eingestellt wird.

13. Verfahren nach Anspruch 12, **dadurch gekennzeichnet, dass** der eingestellte Unterdruck mit Hilfe eines Drucksensors (10) des Analysators überwacht wird.

14. Verfahren nach einem der Ansprüche 11 bis 13, **dadurch gekennzeichnet, dass** die Gewinnung von Plasma bei vorzeitigem Druckanstieg in der Filtereinrichtung (1) automatisch beendet wird, um eine Hämolysierung zu vermeiden.
